# EUROPÄISCHE PATENTANMELDUNG

(11) **EP 1 598 083 A1**
(43) Veröffentlichungstag der Anmeldung: **23.11.2005**
(21) Anmeldenummer: 05009713.8
(22) Anmeldetag: 03.05.2005
(51) Int. Cl.: A61L 9/04, A61L 2/18, A61K 31/327, C11D 3/39, A01N 31/02, C07C 409/24

(54) **Desinfektionsmittel-Zusammensetzung enthaltend zumindest eine Persäure-Verbindung und deren Verwendung zur Raum- und Luftdesinfektion**

(30) Priorität: 06.05.2004 DE 102004022392
(71) Anmelder: Gerhard Ruff GmbH, 87700 Memmingen (DE)
(72) Erfinder: Dückminor, Harald, 87700 Memmingen (DE); Krämer, Albrecht, Dr., 68782 Brühl (DE); Wanke, Erich, 87734 Benningen (DE); Birker, Uwe, 87439 Kempten (DE)
(74) Vertreter: Müller, Hans-Jürgen

(57) **Zusammenfassung**

Die Erfindung betrifft eine Desinfektionsmittel-Zusammensetzung enthaltend eine Percarbonsäure-Verbindung, ein nichtionisches Tensid, ein Puffer-System und Wasser sowie deren Verwendung zur Raum- und Luftdesinfektion.

## Beschreibung

Gegenstand der vorliegenden Erfindung ist eine Desinfektionsmittel-Zusammensetzung enthaltend mindestens eine Percarbonsäure-Verbindung und deren Verwendung zur Raum- und Luftdesinfektion.

Die Luftdesinfektion ist regelmäßig in der Nahrungsmittel- und in der pharmazeutischen Industrie erforderlich. In der Lebensmittelindustrie werden als Desinfektions-Wirkstoffe häufig Chlor, Peressigsäure (häufig auch Peroxyessigsäure genannt), Wasserstoffperoxid und quaternäre Ammoniumverbindungen (QAV) eingesetzt. Hierzu werden in der Regel konzentrierte Lösungen, enthaltend obige Wirkstoffe, vor Gebrauch mit Wasser verdünnt, um dann für unterschiedlichste Desinfektions-Aufgaben Anwendung zu finden.

Nach dem Stand der Technik werden Behälter und Anlagenteile, die bei der Produktion mit Lebensmitteln in Berührung gelangen, durch Spülen mit wässrigen Peroxidlösungen desinfiziert, speziell mit Wasserstoffperoxid und/oder Peressigsäure-Lösungen. Besonders Peressigsäure wird in solchen, üblicherweise geschlossenen, Systemen bevorzugt verwendet, da sie rasch wirkt und ein breites Wirkungsspektrum besitzt. Daneben werden aktivchlorhaltige Produkte für die Desinfektion eingesetzt, da auch hier eine rasche und umfassende Wirkung erwartet werden kann. Für spezielle Anwendungen, wie die aseptische Abfüllung, kommt Wasserstoffperoxid bei erhöhten Konzentrationen bzw. Temperaturen zum Einsatz.

Offene Flächen, Außenseiten von Anlagen und Wände werden im Schaumreinigungs-Verfahren gereinigt und zum Teil im selben Arbeitsgang bzw. in einem separaten zweiten Schritt desinfiziert. Als Wirkstoffe kommen hier meist Aktivchlor sowie quaternäre Ammoniumverbindungen zum Einsatz, wobei jedoch bei Aktivchlor der Geruch häufig als sehr unangenehm empfunden wird.

Zur Raum- und Luftdesinfektion werden wässrige Desinfektionsmittellösungen vernebelt oder auf anderem Weg im Raum verteilt. Dabei steht nur eine beschränkte Auswahl an Wirkstoffen zur Verfügung. Chlor sowie Peressigsäure, als besonders rasch und breit wirksame Desinfektionsmittel, besitzen einen unangenehmen Geruch und zudem korrosive Eigenschaften gegenüber Materialien, auf denen sie sich niederschlagen können. Wasserstoffperoxid ist zwar geruchsneutral, jedoch nur unzureichend wirksam und meistens sauer stabilisiert, so dass auch hier die Gefahr von Korrosion besteht. In der Vergangenheit eingesetzte Aldehyde wie Formaldehyd und Glutardialdehyd sind aufgrund der gesundheitsschädlichen Wirkung inzwischen für die Raumdesinfektion nicht mehr erlaubt. Daher werden üblicherweise verdünnte Lösungen mit quaternären Ammonium-Verbindungen verwendet, die jedoch durch ihre hohe Affinität zu Oberflächen, selbst nach Spülen mit Trinkwasser, Rückstände hinterlassen können, welche z. B. bei der Reifung von Käse in Reiferäumen zu Fehlreifungen führen können.

In der WO 95/34537 bzw. in der WO 99/67213 sind oxidativ wirkende Percarbonsäure-Verbindungen beschrieben, die im Vergleich zu z.B. Peressigsäure keinen unangenehm stechenden Geruch aufweisen und eine vergleichbare Wirksamkeit besitzen. Weiterhin zerfallen die beschriebenen Substanzen (wie auch z. B. Peressigsäure) nach der Anwendung in unproblematische Zersetzungsprodukte.

Es besteht das Bedürfnis oxidativ wirkende Zusammensetzungen, die Oberflächen gut benetzen, sowie eine reduzierte korrosionsauslösende Wirkung in Bezug auf Stahloberflächen aufweisen, zur Verfügung zu stellen. Die Desinfektionsmittel-Zusammensetzung soll bakterizid, fungizid und viruzid wirken.

Obige Aufgabe wird durch nachfolgend erläuterte Erfindung gelöst. Gegenstand der Erfindung ist die Verwendung einer Desinfektionsmittel-Zusammensetzung, zur Raum- und Luftdesinfektion enthaltend mindestens eine Percarbonsäure-Verbindung, wobei die Percarbonsäure-Verbindung eine Dicarbonsäure bzw. deren Derivat mit zumindest einer Persäure-Gruppe (C(=O)-O-O-) ist, ein nichtionisches Tensid, ein Puffer-System sowie Wasser und einen pH-Wert von 5 bis 9, bevorzugt 6 bis 8 aufweist. Gegenstand der Erfindung ist weiterhin eine Zusammensetzung gemäß weiterem unabhängigen Anspruch.

Es ist bevorzugt, dass die erfindungsgemäß eingesetzte Desinfektionsmittel-Zusammensetzung die Komponenten (a) bis (c) in folgenden Mengen enthält:
(a) 0,01 bis 10 Gew.-%, besonders bevorzugt 0,02 bis 3 Gew.-% der Percarbonsäure -Verbindung(en),
(b) 0,1 bis 15 Gew.-%, besonders bevorzugt 0,2 bis 3 Gew.-% eines Puffers,
(c) 0,0001 bis 1 Gew.-%, besonders bevorzugt 0,001 bis 0,2 Gew.-% des nichtionischen Tensids,
neben ggf. weiteren fakultativen Komponenten. Die Differenz zu 100 Gew.-% ist Wasser.

Die weiterhin beanspruchte erfindungsgemäße Desinfektionsmittel- Zusammensetzung ist durch folgende Komponenten gekennzeichnet:
- 0,01 bis 10 Gew.-%, besonders bevorzugt 0,02 bis 3 Gew.-%, einer oder mehrere Percarbonsäure-Verbindungen der allgemeinen Formel I

   R²-O₂C-X-CO₃H,

   worin R² Wasserstoff oder eine Alkylgruppe mit 1 bis 4 Kohlenstoffatomen und X eine Alkylengruppe mit 1 bis 6 Kohlenstoffatomen ist,
- 0,1 bis 15 Gew.-%, besonders bevorzugt 0,2 bis 3 Gew.-%, eines Puffers, und
- 0,0001 bis 1 Gew.-%, besonders bevorzugt 0,001 bis 0,2 Gew.-%, eines nichtionischen Tensids,
- neben ggf. weiteren anderen Bestandteilen und
- zum verbleibenden Rest Wasser,
wobei die Zusammensetzung einen pH-Wert von 5 bis 9 hat.

Die Herabsetzung der Oberflächenspannung führt zu einer bedeutenden Steigerung der Wirksamkeit des Desinfektionsmittels.

Die Regulierung des pH-Wertes in den neutralen Bereich führt zu einer Reduzierung der Korrosivität. Dies geschieht durch Einsatz eines Puffersystems, welches den pH-Wert in einem wählbaren Bereich stabil hält.

Bevorzugt wird die erfindungsgemäße Desinfektionsmittel-Zusammensetzung als Mehrkomponenten-System, besonders bevorzugt als Zweikomponenten-System, in den Handel gebracht, bestehend zumindest aus einer Grundkomponente und einer Aktivatorkomponente enthaltend Komponente (a).

Die Grundkomponente enthält Wasser, das nichtionische Tensid und den Puffer. Die Aktivatorkomponente enthält die Percarbonsäure-Verbindung(en) verdünnt mit Wasser und gegebenenfalls einen Stabilisator zur Lagerstabilisierung der Percarbonsäure-Verbindung(en).

Grundkomponente und Aktivatorkomponente können im Verhältnis 20 zu 1 bis 1000 zu 1, bevorzugt 40 zu 1 bis 500 zu 1, gemischt die Desinfektionsmittel-Zusammensetzung bilden. Die Zusammensetzung kann so durch Vermischen der Komponenten als gebrauchsfertige Lösung ohne Zusatz von Wasser erhalten und eingesetzt werden.

Als Percarbonsäure-Verbindung kommen insbesondere eine oder mehrere Percarbonsäure-Verbindungen der allgemeinen Formel I

R²-O₂C-X -CO₃H I

zum Einsatz, worin R² Wasserstoff oder bevorzugt eine Alkylgruppe mit 1 bis 4 Kohlenstoffatomen und X eine Alkylengruppe mit 1 bis 6 Kohlenstoffatomen ist und insbesondere (CH₂)ₙ mit n von 1 bis 4 ist. Bevorzugt ist, dass als Percarbonsäure laut obiger allgemeiner Formel bzw. deren Derivate (einschließlich Ester und Anhydride) zum Einsatz kommen, bei denen R² eine Ethylgruppe bzw. insbesondere eine Methylgruppe ist.

Es ist besonders bevorzugt, dass eine oder mehrere Percarbonsäure-Verbindungen ausgewählt aus Perbernsteinsäure, Perglutarsäure, Peradipinsäure, Persuccinsäure, Perbernsteinsäure-monomethylester, Perglutarsäuremonomethylester, Peradipinsäuremonomethylester und Persuccinsäuremonomethylester bzw. deren Derivate zum Einsatz kommen. Obige Verbindungen weisen bevorzugt nur eine Persäure-Gruppe (C(=O)-O-O-) auf.

Als nichtionisches Tensid wird insbesondere eine Verbindung ausgewählt aus Alkylpolyglucosiden, Fettalkoholalkoxylaten, Fettaminalkoxylaten, Fettsäurepolyglykolester und/oder Fettsäurepolyglykolamiden eingesetzt. Bevorzugt werden Fettalkoholalkoxylate aufweisend eine Alkoholgruppe mit mindestens 8 Kohlenstoffatomen und zumindest zwei C₂- bis C₄-Alkoxylat-Gruppen, insbesondere gemischte Ethoxylate/Propoxylate und besonders bevorzugt solche der allgemeinen Formel II

R-O-(EO)ₘ-(PO)ₙ-H II

mit Blockstruktur der Alkoxylat-Gruppen, worin R für einen C₈- bis C₁₆-Kohlenwasserstoff, insbesondere für einen C₁₀- bis C₁₄- Kohlenwasserstoff, und n und m (bzw. n1, n2 usw. und m1, m2 usw.) unabhängig voneinander für eine ganze Zahl zwischen 2 und 8 stehen. Die Formel R-O-(EO)ₘ-(PO)ₙ-H umfasst auch andere EO/PO-Blockstrukturen wie beispielsweise R-O- (PO)ₙ- (EO)ₘ-H oder R-O-(PO)ₙ₁-(EO)ₘ₁-(PO)ₙ₂- (EO)ₘ₂-H oder R-O-(EO)ₘ₁-(PO)ₙ- (EO)ₘ₂-H.

Pufferlösungen sind in der Lage, den pH-Wert in einem gewählten Bereich bei Zugabe von Säure oder Lauge stabil zu halten. Puffersubstanzen umfassen Phosphate, Borate, Acetate, Carbonate, Phthalate und deren Mischungen, insbesondere deren Alkalimetallsalze. Die Verwendung von Phosphaten ist bevorzugt.

Vorzugsweise ist der Puffer ein monobasisches, dibasisches und/oder tribasisches Phosphatsalz, gegebenenfalls auch als Hydrat, insbesondere Dinatriumhydrogenphoshat.

Die Desinfektionsmittel-Zusammensetzung soll einen pH-Wert im neutralen Bereich besitzen. Bevorzugt ist ein pH-Wert größer 5 bis 9, besonders bevorzugt größer 6 bis 8.

Die erfindungsgemäße Desinfektionsmittel-Zusammensetzung wirkt abtötend auf Bakterien, Pilze und Viren. Bevorzugt ist die Verwendung zur Raumdesinfektion sowie zur Desinfektion der im Raum befindlichen Luft.

Die aus zwei Komponenten gemischte gebrauchsfertige Lösung des Desinfektionsmittels wird üblicherweise mittels eines Kaltvernebelungsgeräts in Form eines Tröpfchennebels im Raum verteilt. Weiterhin können andere technische Lösungen zur Verteilung der Anwendungslösung im Raum zur Anwendung kommen, wie z. B. durch Zugabe in das luftführende System einer Klimaanlage oder mittels eines Ultraschallzerstäubers.

Die Desinfektionsmittel-Zusammensetzung kann für die Desinfektion von Reiferäumen, Gewächshäusern, Packmittellagern und anderen Räumen zur Desinfektion der im Raum befindlichen Luft eingesetzt werden.

Die nachstehenden Beispiele verdeutlichen die Erfindung.

### Beispiele

In Versuchen wird die Wirksamkeit einer Desinfektionsmittel-Zusammensetzung D1 sowie einer Vergleichsrezeptur V untersucht. Die Inhaltsstoffe der Formulierungen sind in Tabelle 1 aufgeführt.

**Tabelle 1**

| Formulierung V und D1 | | |
|---|---|---|
| | **V** | **D1** |
| Wasser | 98,9 | 97,995 |
| Natriumhydroxid | 0,1 | - |
| Dinatriumhydrogenphosphat | - | 1,000 |
| Fettalkohol, ethoxyliert, propoxyliert | - | 0,005 |
| Esterpersäure-Verbindung gemäß WO 99/67213, Beispiel 4 | 1,0 | 1,000 |

Mit der Vergleichsrezeptur V wurde ein Desinfektions-Versuch durchgeführt, indem im vorgereinigten Raum vor und nach der Desinfektion Luftplatten ausgelegt sowie Abstriche der Wände gemacht wurden. Die Ergebnisse sind in der Tabelle 2 zusammengefasst.

**Tabelle 2**

| Ergebnisse des Versuchs mit Vergleichsrezeptur V, Wachstum auf Platten | | |
|---|---|---|
| | Luft | Wand |
| vor Desinfektion | + | + |
| nach Desinfektion | - | + |

Mit Rezeptur D1 wurde ein Desinfektions-Versuch durchgeführt, indem im vorgereinigten Raum vor und nach der Desinfektion Kolonie-Zahlen bestimmt wurden. Die Ergebnisse sind in Tabelle 3 aufgeführt.

**Tabelle 3**

| Ergebnisse des Versuchs mit D1, Kolonie-Zahlen | | | | | |
|---|---|---|---|---|---|
| | | Hefen | Schimmel | Proteolyten | Coli |
| Luft | vor Desinfektion | 12000 | 138000 | 4000 | <10 |
| | nach Desinfektion | <1000 | <10 | <10 | <10 |
| Wand | vor Desinfektion | 179000 | 2500000 | 27000 | <100 |
| | nach Desinfektion | <1000 | <100 | <10 | <10 |

Es zeigt sich, dass die erfindungsgemäßen Desinfektionsmittel-Zusammensetzungen für den vorgesehenen Einsatzzweck geeignet sind und die gewünschte Wirksamkeit aufweisen.

## Patentansprüche

1. Verwendung einer Desinfektionsmittel-Zusammensetzung zur Raum- und Luftdesinfektion mittels Ausbringen eines Sprühnebels, **dadurch gekennzeichnet, dass** die Zusammensetzung mindestens eine Percarbonsäure-Verbindung, ein nichtionisches Tensid, ein Puffer-System und Wasser enthält, wobei die Zusammensetzung einen pH-Wert von 5 bis 9 hat und die Percarbonsäure-Verbindung eine Dicarbonsäure-Verbindung bzw. deren Derivat ist, die zumindest eine Persäure-Gruppe (-C(=O)-O-O-) aufweist.

2. Verwendung nach Anspruch 1, **dadurch gekennzeichnet, dass** die mindestens eine Percarbonsäure-Verbindung pro Molekül eine Persäure-Gruppe (-C(=O)-O-O-) und eine Estergruppe (-C(=O)-O-R²) aufweist und R² für eine Alkylgruppe mit 1 bis 4 Kohlenstoffatomen, insbesondere Methyl oder Ethyl, steht.

3. Verwendung nach Anspruch 1, **dadurch gekennzeichnet, dass** die Zusammensetzung folgende Komponenten enthält:
- 0,01 bis 10 Gew.-%, besonders bevorzugt 0,02 bis 3 Gew.-% , eine oder mehrere Percarbonsäure-Verbindungen der allgemeinen Formel I
R²-O₂C-X -CO₃H I
worin R² Wasserstoff oder eine Alkylgruppe mit 1 bis 4 Kohlenstoffatomen und X eine Alkylengruppe mit 1 bis 6 Kohlenstoffatomen ist und insbesondere (CH₂)ₙ mit n von 1 bis 4,
- 0,1 bis 15 Gew.-%, besonders bevorzugt 0,2 bis 3 Gew.-% des Puffers, und
- 0,0001 bis 1 Gew.-%, besonders bevorzugt 0,001 bis 0,2 Gew.-% des nichtionischen Tensids,
- neben ggf. weiteren anderen Bestandteilen und
- zum verbleibenden Rest Wasser.

4. Verwendung nach Anspruch 3, **dadurch gekennzeichnet, dass** als Percarbonsäure -Verbindungen Verbindungen nach Formel I zum Einsatz kommen, bei denen R² Wasserstoff und/oder eine Methyl- oder Ethylgruppe ist.

5. Verwendung nach zumindest einem der Ansprüche 1 bis 4, **dadurch gekennzeichnet, dass** als Percarbonsäure-Verbindung eine oder mehrere der folgenden Säuren bzw. Ester bzw. deren Derivate eingesetzt werden:
Perbernsteinsäure, Perglutarsäure, Peradipinsäure, Persuccinsäure, Perbern-steinsäuremonomethylester, Perglutarsäuremonomethylester, Peradipinsäuremonomethylester und Persuccinsäuremonomethylester.

6. Verwendung nach zumindest einem der vorhergehenden Ansprüche, **dadurch gekennzeichnet, dass** als nichtionisches Tensid eine Verbindung ausgewählt aus Alkylpolyglucosiden, Fettalkoholalkoxylaten, Fettaminalkoxylaten, Fettsäureolyglykolester, Fettsäurepolyglykolamiden alleine oder in Mischung eingesetzt wird, insbesondere von Fettalkoholalkoxylaten.

7. Verwendung nach zumindest einem der vorhergehenden Ansprüche, **dadurch gekennzeichnet, dass** als nichtionisches Tensid ein Fettalkoholalkoxylat der allgemeinen Formel II
R-O-(EO)ₘ-(PO)ₙ-H II
verwendet wird, worin R für einen C₈- bis C₁₆-Kohlenwasserstoff, insbesondere für einen C₁₀- bis C₁₄-Kohlenwasserstoff, und n und m unabhängig voneinander für eine ganze Zahl zwischen 2 und 8 stehen.

8. Verwendung nach zumindest einem der vorhergehenden Ansprüche, **dadurch gekennzeichnet, dass** als Puffersystem ein Phosphat-Puffer, Borat-Puffer, Acetat-Puffer, Carbonat-Puffer, Phthalat-Puffer, insbesondere in Form deren Alkalimetallsalzen, sowie deren Mischungen verwendet wird.

9. Verwendung nach zumindest einem der vorhergehenden Ansprüche, **dadurch gekennzeichnet, dass** das Puffersystem Dinatriumhydrogenphosphat umfasst.

10. Verwendung nach zumindest einem der vorhergehenden Ansprüche, **dadurch gekennzeichnet, dass** die Zusammensetzung als Mehrkomponenten-System, bevorzugt als Zweikomponenten-System, zur Verwendung bereitgestellt wird, bestehend zumindest aus einer Grundkomponente, enthaltend Wasser, das nichtionische Tensid und den Puffer, und einer Aktivatorkomponente, enthaltend die Percarbonsäure-Verbindung(en) verdünnt mit Wasser sowie gegebenenfalls einen Stabilisator für peroxidische Verbindungen.

11. Verwendung nach Anspruch 10, **dadurch gekennzeichnet, dass** die Grundkomponente und die Aktivatorkomponente im Mischungsverhältnis 20 zu 1 bis 1000 zu 1, bevorzugt 40 zu 1 bis 500 zu 1 die Desinfektionsmittel-Zusammensetzung bilden.

12. Verwendung nach Anspruch 10 oder 11, **dadurch gekennzeichnet, dass** diese durch Vermischen der Komponenten als gebrauchsfertige Lösung ohne Zusatz von Wasser erhalten und eingesetzt wird.

13. Verwendung gemäß einem der vorhergehenden Ansprüche, **dadurch gekennzeichnet, dass** die Desinfektionsmittel-zusammensetzung in Räumen eingesetzt wird, die der Herstellung, und/oder Aufbewahrung von Lebensmitteln, Pflanzen und/oder Saatgut, oder von Verpackungen für Lebensmittel dienen.

14. Desinfektionsmittel-Zusammensetzung enthaltend folgende Komponenten:
- 0,01 bis 10 Gew.-% einer oder mehrere Percarbonsäure-Verbindungen der allgemeinen Formel I
R²-O₂C-X-CO₃H,
worin R² Wasserstoff oder eine Alkylgruppe mit 1 bis 4 Kohlenstoffatomen und X eine Alkylengruppe mit 1 bis 6 Kohlenstoffatomen ist,
- 0,1 bis 15 Gew.-% eines Puffers, und
- 0,0001 bis 1 Gew.-% eines nichtionischen Tensids,
- neben ggf. weiteren anderen Bestandteilen und
- zum verbleibenden Rest Wasser,
wobei die Zusammensetzung einen pH-Wert von 5 bis 9 hat.

15. Desinfektionsmittel-Zusammensetzung gemäß Anspruch 14, **dadurch gekennzeichnet, dass** das R² für eine Alkylgruppe mit 1 bis 4 Kohlenstoffatomen, insbesondere Methyl oder Ethyl steht.

16. Desinfektionsmittel-Zusammensetzung gemäß Anspruch 14, **dadurch gekennzeichnet, dass** das nichtionische Tensid ein Fettalkoholalkoxylat ist, vorzugsweise ein Fettalkoholalkoxylat der allgemeinen Formel II
R-O-(EO)ₘ-(PO)ₙ-H II
worin R für einen C₈- bis C₁₆-Kohlenwasserstoff, insbesondere für einen C₁₀- bis C₁₉-Kohlenwasserstoff, und n und m unabhängig voneinander für eine ganze Zahl zwischen 2 und 8 stehen.

17. Desinfektionsmittel- Zusammensetzung gemäß Anspruch 14, **dadurch gekennzeichnet, dass** der Puffer ein Phosphat-Puffer, Borat-Puffer, Acetat-Puffer, Carbonat-Puffer, Phthalat-Puffer, sowie deren Mischungen ist, vorzugsweise ein Hydrogenphosphat-Puffer und insbesondere in Form deren Alkalimetallsalzen.
